(19) Europäisches Patentamt
European Patent Office
Office européen des brevets

(11) EP 0 648 727 B1

(12) FASCICULE DE BREVET EUROPEEN

(45) Date de publication et mention de la délivrance du brevet:
**27.08.1997 Bulletin 1997/35**

(51) Int Cl.$^6$: **C07C 19/08**, C07C 17/395

(21) Numéro de dépôt: **94402264.9**

(22) Date de dépôt: **10.10.1994**

# (54) Procédé de purification du 1,1,1,2-tétrafluoroéthane

Verfahren zur Reinigung von 1,1,1,2-Tetrafluorethan

Process for the purification of 1,1,1,2-Tetrafluorethane

(84) Etats contractants désignés:
**DE ES FR GB GR IT NL**

(30) Priorité: **13.10.1993 FR 9312190**
**22.06.1994 FR 9407642**

(43) Date de publication de la demande:
**19.04.1995 Bulletin 1995/16**

(73) Titulaire: **ELF ATOCHEM S.A.**
**92800 Puteaux (FR)**

(72) Inventeurs:
- **Cheminal, Bernard**
  **F-69510 Saucieu-en-Jarrest (FR)**
- **Lantz, André**
  **F-69390 Vernaison (FR)**

(74) Mandataire: **Leboulenger, Jean et al**
**Elf Atochem S.A.,**
**Département Propriété Industrielle,**
**Cedex 42 - La Défense 10**
**92091 Paris la Défense (FR)**

(56) Documents cités:
**EP-A- 0 420 709** **EP-A- 0 548 742**

- **DATABASE WPI Week 9311, Derwent Publications Ltd., London, GB; AN 93-088600 & JP-A-5 032 567**
- **DATABASE WPI Section Ch, Week 9252, Derwent Publications Ltd., London, GB; Class E16, AN 92-427160 & JP-A-4 321 632**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 648 727 B1

Printed by Jouve, 75001 PARIS (FR)

## Description

La présente invention concerne le domaine des hydrocarbures fluorés et a plus particulièrement pour objet la purification du 1,1,1,2-tétrafluoroéthane.

Ce composé, connu dans le métier sous la désignation F134a, est notamment destiné à remplacer le dichlorodifluorométhane (F12) actuellement utilisé comme fluide frigorifique, mais suspecté de contribuer à l'affaiblissement de la couche d'ozone stratosphérique. Pour ce faire, le F134a doit satisfaire à des normes de qualité relativement à la présence d'impuretés a priori toxiques comme les oléfines chlorofluorées.

Or, l'une des synthèses industrielle du F134a consiste en une fluoration catalytique en phase gazeuse du trichloréthylène ou du 1-chloro-2,2,2-trifluoroéthane (F133a) qui sous-produit toujours des quantités variables de 1-chloro-2,2,-difluoroéthylène (F1122) qui, vu son point d'ébullition (-17,7°C), s'avère très difficile à éliminer complètement du F134a (pEb. = -26,5°C) par simple distillation, surtout sous pression.

Le F134a obtenu selon ce procédé ou selon d'autres procédés contient en général encore d'autres composés oléfiniques tels que des butènes ou des propènes fluorés. Les oléfines en $C_4$ telles que $CF_3CF = CF\text{-}CF_3$ (F1318), $CF_3CF = CHCF_3$ (F1327), $CF_3CH = CClCF_3$ (F1326) et $CF_3CH = CHCF_3$ (F1336) ne sont pas particulièrement gênantes car elles peuvent être séparées du F134a par distillation. Les F134a obtenus industriellement, et en particulier celui obtenu par fluoration en phase gazeuse du trichloroéthylène ou du F133a, contiennent en général des quantités plus ou moins importantes de polyfluoropropènes tels que $CF_3CH = CH_2$ (1243), $CF_3CF = CH_2$ (1234), $CF_3CF = CHF$ (1225) ou leurs isomères qui sont très difficiles à séparer du F134a par distillation car leurs points d'ébullition sont très proches de celui du 134a.

De nombreux procédés de purification du F134a , et en particulier d'élimination du F1122 dans le F134a, ont déjà été proposés. On peut ainsi citer :

- l'hydrogénation catalytique du F1122 et/ou d'autres oléfines fluorées (WO 9008750, JP 02273634, JP 04095037) ;
- l'adsorption des impuretés sur charbon actif (EP 389334) ou sur tamis moléculaire (US 4906796, JP 03072437, EP 503796, EP 511612, EP 526002) ;
- l'oxydation du F1122 par le permanganate de potassium aqueux (US 4129603).

Aucun de ces procédés n'est entièrement satisfaisant du point de vue industriel. Ainsi, le traitement au permanganate aqueux nécessite un séchage du F134a après la purification qui augmente considérablement le coût de ce traitement. L'adsorption physique sur charbon ou tamis moléculaire ne peut être envisagée industriellement que pour un traitement de finition car, compte-tenu des capacités d'adsorption des matériaux proposés, il paraît tout-à-fait inéconomique de traiter des produits contenant plus que quelques dizaines de ppm d'impuretés adsorbables. Par ailleurs, d'après les documents cités ci-dessus, ces techniques d'adsorption ne permettent d'éliminer que le F1122 et pas les oléfines en $C_3$ ou $C_4$. L'hydrogénation catalytique nécessite des installations spéciales (compatibles avec l'hydrogène) qui ne sont industriellement envisageables que si le F134a a lui-même déjà été obtenu selon un procédé d'hydrogénolyse.

D'autres procédés proposés, tels que la fluoration des oléfines par du fluor élémentaire (EP 548744), sont tout aussi inintéressants industriellement.

Plus intéressante est la technique décrite dans le brevet US 4158675 qui concerne un procédé d'épuration du F1122 consistant à faire réagir les gaz issus de la réaction principale :

$$\text{F133a} + \text{HF} \rightleftharpoons \text{F134a} + \text{HCl}$$

sans séparation de l'HCl, de l'HF ou du F133a non transformés, dans un second réacteur maintenu à une température plus basse que celle de la réaction principale. A partir d'un mélange gazeux dont la teneur en F1122, ramenée aux composés organiques, est de 5300 vpm (volume par million), le traitement en ligne à 160°C conduit à un taux de 7 vpm en F1122.

Dans ce procédé, l'impureté éliminée (F1122) est refluorée en F133a c'est-à-dire en un produit recyclable. Mais l'inconvénient majeur de ce procédé réside dans la nécessité d'avoir à traiter un débit de gaz important et donc d'aboutir à un volume réactionnel élevé, ce qui entraîne un investissement et un coût d'entretien prohibitifs. Par ailleurs, le procédé ne concerne aussi que l'élimination du F1122.

Pour éviter ces inconvénients, on a proposé de traiter en phase gazeuse, en l'absence d'acide chlorhydrique, un mélange gazeux de F134a brut et d'HF en présence d'un catalyseur de fluoration (JP 04321632, EP 548742 et demande FR 9209700). Durant ce traitement, l'acide fluorhydrique s'additionne sur le F1122 et certains autres oléfines (chloro) fluorées telles que $CF_2 = CFH$ (F1123), $CF_3CH = CHCF_3$ (F1336) et les transforme en composés saturés plus faciles

à séparer et/ou à recycler par distillation. Ce procédé est particulièrement élégant, mais on a malheureusement constaté que, si le F1122 est particulièrement facile à éliminer selon cette méthode, d'autres oléfines fluorées telles que, par exemple, les fluoropropènes F1243, 1234 et 1225, sont très peu réactives dans ces conditions et ne peuvent pas être entièrement éliminées selon ce procédé.

On a par ailleurs proposé de purifier le F134a par chloration du F1122 et éventuellement d'autres impuretés oléfiniques. Cette chloration peut être réalisée en phase gazeuse soit par initiation photochimique (WO 93/12058), soit par initiation thermique en présence d'un catalyseur de chloration comme un charbon actif ou une alumine (JP 05032567). Au cours de ce traitement de chloration, l'oléfine la plus importante (le F1122) est transformée en F122 :

$$CF_2 = CHCl + Cl_2 \longrightarrow CF_2Cl - CHCl_2$$

F1122 F122

et est donc perdue. Ces procédés de chloration ne sont pas très sélectifs et donnent lieu à une sous-production de $CF_3CHFCl$ (F124) par chloration du F134a ce qui diminue d'autant le rendement et l'intérêt de cette purification.

Pour éviter les inconvénients des techniques précitées, la présente invention propose un moyen particulièrement efficace et économique pour purifier un F134a brut contenant des impuretés insaturées.

Le procédé selon l'invention consiste à traiter en phase gazeuse, un mélange gazeux de F134a brut, d'HF et de chlore à une température comprise entre 100 et 300°C et sous une pression allant de la pression atmosphérique jusqu'à 2,5 MPa, en présence d'un catalyseur de fluoration, le rapport molaire HF/F134a étant compris entre 0,05 et 0,5 et le rapport molaire $Cl_2$/F134a étant compris entre 0,0001 et 0,1.

Dans un F134a brut, le taux d'impuretés oléfiniques peut varier entre 50 et 15000 ppm (0,005 à 1,5 %) par rapport au F134a et est le plus souvent compris entre 500 et 5000 ppm (0,05 à 0,5 %). A côté des oléfines (chloro)fluorées, le F134a brut peut également contenir des quantités variables d'autres composés comme, par exemple, le F133a (0 à 7 %), le 1,1,1-trifluoroéthane (F143a), le monochlorotrifluoroéthane (F124) et le pentafluoroéthane (F125) ; la présence de ces impuretés saturées ne nuit nullement à l'efficacité du procédé selon l'invention.

Parmi les impuretés oléfiniques (chloro)fluorées présentes dans le F134a brut, le F1122 est en général l'impureté la plus importante. Les éventuelles autres impuretés oléfiniques telles que les F1123, F1243, F1234, F1225, F1318, F1327, F1326 et F1336 sont soit inexistantes soit généralement présentes à des teneurs plus faibles (10 à 500 ppm) ; parmi ces dernières, les plus gênantes sont les différents F1243, F1234 et F1225.

Grâce à l'utilisation conjointe d'HF et de $Cl_2$, le procédé selon l'invention permet d'éliminer pratiquement quantitativement la plupart des oléfines (chloro)fluorées. Il permet de transformer entièrement non seulement le F1122, mais aussi les oléfines en $C_3$ (F1243, F1234, F1225, ...) particulièrement difficiles à éliminer par traitement avec de l'acide fluorhydrique seul.

Dans le procédé selon l'invention, les oléfines (chloro)fluorées telles que les F1122, 1123, 1243, 1234, 1225, 1318, 1327, 1336,... peuvent réagir soit avec l'HF, soit avec le chlore. Le F1122 qui est l'impureté principale peut ainsi se transformer en F133a et en F122 :

HF $CF_3CH_2Cl$ F133a

$CF_2 = CHCl$

$Cl_2$ $CF_2Cl\text{-}CHCl_2$ F122

Le F133a étant directement recyclable au réacteur de fluoration, on a bien évidemment intérêt à privilégier la première réaction. Compte-tenu des différences de réactivité avec l'HF du F1122 et des autres oléfines présentes dans le F134a brut, il a pu être montré qu'un bon choix des conditions opératoires permet de minimiser la sous-production du F122 tout en transformant quantitativement aussi bien le F1122 que les autres oléfines comme les F1243, F1234 et F1225. La fluoration du F1122 en F133a permet ainsi de transformer cette impureté en un produit recyclable au réacteur mais cette prépondérance de la fluoration sur la chloration du F1122 ne limite pas l'invention car, dans le cas de faibles quantités de F1122, la perte par chloration en F122 n'est pas importante.

Lors de la mise en oeuvre du procédé selon l'invention, le chlore peut réagir avec le F134a ou avec certaines

impuretés saturées telles que le F133a pour fournir des produits de chloration selon les réactions suivantes :

$$\underset{\text{F134a}}{CF_3\text{-}CH_2F} + Cl_2 \rightarrow \underset{\text{F124}}{CF_3CHFCl} + HCl$$

$$\underset{\text{F133a}}{CF_3\text{-}CH_2Cl} + Cl_2 \rightarrow \underset{\text{F123}}{CF_3CHCl_2} + HCl$$

Bien que ces produits de chloration ne soient pas gênants et peuvent être séparés du F134a par distillation, on a néanmoins intérêt à minimiser ces réactions secondaires car elles se traduisent par un perte de rendement. Par ailleurs, ces réactions de chloration libèrent de l'acide chlorhydrique qui peut, en présence du catalyseur de fluoration, transformer une petite partie du F134a en F133a selon la réaction inverse de celle de la fabrication du F134a :

$$\underset{\text{F134a}}{CF_3CH_2F} + HCl \rightleftharpoons \underset{\text{F133a}}{CF_3CH_2Cl} + HF$$

En choisissant judicieusement les conditions opératoires (température, temps de contact, rapports molaires $Cl_2$/F134a et HF/F134a), il est possible de minimiser ces réactions de substitution et de ne faire réagir le chlore pratiquement qu'avec les oléfines. Le choix de la température est particulièrement important pour l'obtention d'une réaction sélective sans sous-production de produits de chloration du F134a ou éventuellement du F133a. Cependant, la portée de la présente invention ne saurait être limitée à l'obtention d'une bonne sélectivité car les F123 et F124 éventuellement sous-produits sont des HCFC, facilement séparables du F134a et utilisables comme substituts aux CFC. Une perte de F134a ou éventuellement de F133a n'est donc pas obligatoirement un problème important.

Le traitement catalytique en phase gazeuse du F134a brut avec de l'HF et du $Cl_2$ selon l'invention est avantageusement réalisé à une température comprise entre 100 et 300°C et, de préférence, sous une pression comprise entre la pression atmosphérique et 1,5 MPa.

Le temps de contact peut varier entre 10 et 200 secondes, mais on préfère un temps de contact compris entre 20 et 100 secondes.

Comme indiqué précédemment, le rapport molaire HF/F134a peut varier entre 0,05 et 0,5. On préfère cependant opérer avec un rapport molaire HF/F134a compris entre 0,125 et 0,300 et, plus particulièrement, un rapport molaire proche de celui correspondant à l'azéotrope HF-134a (0,15).

Le rapport molaire $Cl_2$/F134a peut varier entre 0,0001 et 0,1, mais on préfère opérer avec un rapport molaire $Cl_2$/F134a compris entre 0,001 et 0,03.

A l'issue du traitement selon l'invention, le flux gazeux ne contient plus, ou seulement des traces, d'impuretés oléfiniques et peut alors être soumis aux opérations conventionnelles (lavage à l'eau, lavage avec une solution de soude-sulfite de sodium, séchage, distillation ...) pour séparer l'HF et le chlore non transformés et les composés saturés autres que le F134a.

Les catalyseurs de fluoration à utiliser pour la mise en oeuvre du procédé selon l'invention peuvent être des catalyseurs massiques ou des catalyseurs supportés, le support stable dans le milieu réactionnel étant, par exemple, un charbon actif, une alumine fluorée, le fluorure d'aluminium ou le phosphate d'aluminium.

Parmi les catalyseurs massiques, on peut citer plus particulièrement l'oxyde de chrome préparé selon l'une quelconque des méthodes connues de l'homme du métier (procédé sol-gel, précipitation de l'hydroxyde à partir des sels de chrome, réduction de l'anhydride chromique, etc...). Les dérivés de métaux tels que nickel, fer, manganèse, cobalt, zinc, peuvent également convenir seuls ou en association avec le chrome, sous forme de catalyseurs massiques, mais aussi sous forme de catalyseurs supportés.

Les catalyseurs supportés peuvent être employés sous forme de billes, d'extrudés, de pastilles ou même, si l'on opère en lit fixe, sous forme de morceaux. Pour les catalyseurs massiques, la forme de pastilles ou de billes est généralement préférée. Lorsqu'on opère en lit fluide, on préfère utiliser un catalyseur sous forme de billes ou d'extrudés.

Comme exemples non limitatifs de catalyseurs, on peut mentionner :

- les microbilles d'oxyde de chrome obtenues par le procédé sol-gel comme décrit dans le brevet FR 2 501 062,

- les catalyseurs d'oxyde de chrome déposé sur charbon actif (brevet US 4 474 895), sur phosphate d'aluminium (brevet EP 55 958) ou sur fluorure d'aluminium (brevets US 4 579 974 et 4 579 976),
- les catalyseurs mixtes d'oxyde de chrome et de fluorure de nickel déposés sur fluorure d'aluminium (demande de brevet EP 0 486 333),
- les catalyseurs massiques à base d'oxydes de chrome et de nickel (demande de brevet EP 0546883),
- les catalyseurs de fluorure de nickel déposé sur une alumine fluorée.

Les brevets précités dont le contenu est incorporé ici par référence décrivent largement le mode de préparation de ces catalyseurs, mais aussi leur mode d'activation, c'est-à-dire de transformation préalable du catalyseur en espèces actives stables par fluoration au moyen d'HF gazeux dilué par des composés inertes (azote) ou non (air ou 1,1,2-trichloro-1,2,2-trifluoroéthane). Durant cette activation, les oxydes métalliques servant de matière active (par exemple l'oxyde de chrome) ou de support (par exemple l'alumine) peuvent être partiellement ou complètement transformés en fluorures correspondants.

Les exemples suivants illustrent l'invention sans la limiter. Sauf indication contraire, les teneurs (% et ppm) indiquées sont exprimées en volume.

**EXEMPLE 1(Comparatif)**

Cet exemple est destiné à illustrer la réactivité des oléfines F1243, F1234 et F1225 avec de l'HF, sans chlore, en présence d'un catalyseur de fluoration.

Dans un réacteur tubulaire en Inconel 600 de 28 mm de diamètre intérieur et d'un volume de 200 ml, on a placé 100 ml d'un catalyseur à base de fluorure de nickel et d'oxyde de chrome déposés sur fluorure d'aluminium. Les caractéristiques physico-chimiques de ce catalyseur, préparé comme décrit dans la demande de brevet EP 0 486 333 et activé en lit fixe par un mélange azote/HF, sont les suivantes :

- *Composition chimique (pondérale)* :
  - fluor : 58,6 %
  - aluminium : 25,9 %
  - nickel : 6,4 %
  - chrome : 6,0 %
- *Propriétés physiques* :
  - densité apparente (en vrac) : 0,85 g/ml
  - surface BET : 23 $m^2$/g
  - volume des pores d'un rayon compris entre 4 nm et 63 µm : 0,4 ml/g
  - surface des pores d'un rayon supérieur à 4 nm : 23 $m^2$/g

Après une ultime activation "in situ" du catalyseur à l'aide d'un mélange gazeux HF/F133a (rapport molaire : 0,4) entre 25 et 250°C, on a alimenté le réacteur avec un mélange gazeux constitué d'HF et de F134a brut dans des proportions telles que le rapport molaire HF/F134a soit égal à 0,2, c'est-à-dire proche de la composition azéotropique.

Le F134a utilisé contenait 50 ppm de F1243 + F1234 et 14 ppm de F1225. Les F1243 et F1234 n'étaient pas séparés dans les conditions d'analyse (chromatographie en phase gaz) et les 50 ppm correspondent à la somme F1243 + F1234. D'autres analyses du même mélange ont néanmoins montré que la teneur en F1243 était plus importante que celle de F1234.

La pression absolue du réacteur a été fixée à 1,2 MPa et le débit (d) d'alimentation du mélange F134a + HF a été ajusté de façon à voir un temps de contact (t) de 80 secondes, d et t étant liés par la relation :

$$t = \frac{3600 \times V \times 273 \times P \times 10}{22,4 \times d \times (T+273)}$$

où

P = pression en MPa
t = temps de contact en secondes
d = débit en moles/heure
V = volume de catalyseur en vrac, exprimé en litres

T = température du réacteur en degré Celsius

Un échantillon gazeux, débarrassé de l'HF en excès, était analysé par CPV en sortie de réacteur pour suivre l'évolution du taux d'oléfines dans les produits organiques.

Deux essais ont été réalisés respectivement à 250 et 300°C (durée 48 heures) et les résultats suivants ont été obtenus :

| Taux d'oléfines en sortie de réacteur (en ppm) | 250°C | 300°C |
|---|---|---|
| F1243 + F1234 | 30 | 20 |
| F1225 | 12 | 12 |

On a ensuite fait passer dans le même réacteur, sur le même catalyseur, dans les mêmes conditions de pression et de temps de contact, un mélange d'HF et de F134a (rapport molaire HF/F134a : 0,18); le F134a contenait 2400ppm de F1122. Comme ci-dessus deux essais ont été réalisés à 250°C et 300°C, et dans les deux cas, la teneur résiduelle en F1122 a été inférieure à 5 ppm.

Ces essais montrent ainsi très clairement que les oléfines F1234, F1243 et surtout F1225 sont nettement moins réactives vis-à-vis de l'HF que le F1122 et qu'il est pratiquement impossible de les éliminer entièrement.

**EXEMPLE 2**

Dans le même réacteur et avec le même catalyseur qu'à l'exemple comparatif 1, on a fait passer un mélange $Cl_2$/HF/F134a dans les conditions suivantes :

| | |
|---|---|
| Pression | 1,2 MPa |
| Temps de contact | 80 s |
| Rapport molaire HF/F134a | 0,2 |
| Rapport molaire $Cl_2$/F134a | 0,001 à 0,01 |
| Température | 150 à 275°C |

Le F134a contenait 40 ppm de F1243 + F1234 et 5 ppm de F1225.

Les résultats suivants ont été obtenus :

| Température (°C) | Rapport molaire $Cl_2$/F134a | TAUX résiduels d'oléfines (ppm) | | Taux de F133a et F124 formés (%) | |
|---|---|---|---|---|---|
| | | F1243 + F1234 | F1225 | F124 | F133a |
| 275 | 0,01 | 4 | 3 | 0,9 | 0,9 |
| 250 | 0,01 | < 1 | 2 | 0,8 | 0,8 |
| 200 | 0,01 | < 1 | < 1 | 0,02 | 0,005 |
| 150 | 0,01 | < 1 | 3 | 0,002 | 0,0005 |
| 275 | 0,001 | 25 | 4 | 0,08 | 0,09 |

Ces résultats montrent que l'action simultanée du $Cl_2$ et de l'HF permet d'éliminer entièrement les oléfines F1243, F1234 et F1225. Aux températures supérieures à 200°C, l'excès de chlore est consommé pour chlorer un peu de F134a en F124 avec formation simultanée de F133a. A 200°C ou aux températures inférieures à 200°C, la réaction est par contre beaucoup plus sélective et la réaction de chloration du F134a en F124 devient tout-à-fait négligeable.

**EXEMPLE 3**

Dans le même réacteur que celui utilisé précédemment et contenant le même catalyseur, on a fait passer un mélange de F134a-HF-$Cl_2$ dans les conditions suivantes :

| | |
|---|---|
| Pression | 1,2 MPa |

(suite)

| | |
|---|---|
| Temps de contact | 80 s |
| Rapport molaire HF/F134a | 0,2 |
| Rapport molaire $Cl_2$/F134a | 0,005 à 0,02 |
| Température | 150 à 275°C |

Le F134a utilisé était un produit industriel contenant entre autres les impuretés suivantes :

| | |
|---|---|
| F133a | 3,37 % |
| F124 | 1,95 % |
| F1122 | 1370 ppm |
| F1243 + F1234 | 173 ppm |
| F1225 | 393 ppm |

Les résultats suivants ont été obtenus :

| Température (°C) | Rapport molaire $Cl_2$/ F134a | Taux d'oléfines non transformées (ppm) | | | Taux de F122 formé (ppm) |
|---|---|---|---|---|---|
| | | F1243 + F1234 | F1225 | F1122 | |
| 150 | 0,01 | 8 | 12 | 8 | 185 |
| 200 | 0,02 | < 1 | < 1 | 8 | 1200 |
| 200 | 0,01 | < 1 | < 1 | 8 | 750 |
| 200 | 0,005 | 2 | < 1 | 7 | 430 |
| 275 | 0,01 | 39 | 14 | 6 | 156 |

Ces résultats illustrent parfaitement l'excellente réactivité des impuretés oléfiniques avec le mélange $Cl_2$ + HF. Ils montrent aussi qu'une diminution du rapport molaire $Cl_2$/F134a permet de diminuer le taux de F122 formé, même à transformation égale du F1122. Les teneurs en F134a et en F124 n'ont pas bougé de façon significative au cours des essais réalisés à 200°C.

En dehors du F122, on a vu apparaître dans les analyses chromatographiques du produit organique en sortie de réacteur, plusieurs produits lourds nouveaux qui n'ont pas été identifiés mais qui pourraient correspondre aux produits de chloration des oléfines en $C_3$.

## EXEMPLE 4

Dans un réacteur tubulaire en Inconel 600 de 28 mm de diamètre intérieur et d'un volume de 200 ml, on a placé 50 ml d'un catalyseur constitué de microbilles d'oxyde de chrome massique, préparé comme décrit à l'exemple 3 du brevet FR 2 501 062. On a ensuite alimenté ce réacteur catalytique fonctionnant en lit fixe avec un mélange, à l'état gazeux, constitué de F134a brut, d'HF et de chlore dans des proportions telles que le rapport molaire HF/F134a soit égal à 0,25 et que le rapport molaire $Cl_2$/F134a soit égal à 0,01.

Le F134a brut contenait les impuretés suivantes :

| | |
|---|---|
| F124 | 0,5 % |
| F133a | 1,5 % |
| F1122 | 1200 ppm |
| F1243 + F1234 | 55 ppm |
| F1225 | 24 ppm |

La température du réacteur était fixée à 225°C et le débit d'alimentation du mélange a été réglé de façon à avoir un temps de contact de 50 secondes sous une pression de 1,5 MPa.

L'analyse du produit sorti du réacteur, après élimination du chlore et de l'HF en excès, a fourni les résultats

suivants :

| | |
|---|---|
| F1122 | 7 ppm |
| F1243 + F1234 | < 2 ppm |
| F1225 | < 2 ppm |

Les teneurs en F124 et F133a n'ont pas varié de façon significative.

## EXEMPLE 5

### *(a) Préparation et activation d'un catalyseur au nickel sur alumine fluorée*

Dans un évaporateur rotatif, on a placé 500 ml d'alumine partiellement fluorée (contenant globalement 83 % en masse de fluorure d'aluminium et 16 % d'alumine), obtenue dans une étape précédente par fluoration d'alumine GRACE HSA vers 300°C à l'aide d'azote et d'acide fluorhydrique. Ce support fluoré présentait avant imprégnation les caractéristiques physico-chimiques suivantes :

| | |
|---|---|
| forme | billes de 1-2 mm de diamètre |
| densité apparente | 0,57 g/ml |
| surface BET | 67 $m^2/g$ |
| volume poreux | 0,72 ml/g (pour les rayons compris entre 4 nm et 63 µm) |

On a préparé par ailleurs une solution d'imprégnation contenant 39 g de chlorure de nickel hexahydraté et 200 g d'eau, qu'on a ensuite introduite en 45 minutes, à température ambiante et sous pression atmosphérique, sur le support en agitation. Le catalyseur a alors été séché sous courant d'azote, en lit fluidisé, vers 110°C pendant 4 heures, puis chargé dans un réacteur en Inconel 600 et activé en lit fixe par un mélange azote/HF suivant la procédure décrite à l'exemple 1 du brevet EP 0486333. Après ce traitement les caractéristiques physico-chimiques du catalyseur sont les suivantes :

- *Composition chimique (pondérale)* :
  - fluor : 60 %
  - aluminium : 30 %
  - nickel : 2,95 %
- *Propriétés physiques* :
  - densité apparente (en vrac) : 0,66 g/ml
  - surface BET : 28,7 $m^2/g$
  - volume des pores d'un rayon compris entre 4 nm et 63 µm : 0,59 ml/g
  - surface des pores d'un rayon supérieur à 4 nm : 33 $m^2/g$

### *(b) Purification du F134a par HF sans chlore*

Dans un réacteur tubulaire en Inconel 600 de 41 mm de diamètre intérieur et d'un volume de 550 ml, on a placé 300 ml de ce catalyseur, puis on a alimenté le réacteur avec un mélange HF/F134a brut dans des proportions telles que le rapport molaire HF/F134a soit égal à 0,2, c'est-à-dire proche de la composition azéotropique.

Le F134a de départ contenait les impuretés suivantes :

*Impuretés oléfiniques :*

42 ppm de F1243 + F1234 ($C_3H_3F_3$ + $C_3H_2F_4$) non séparés
157 ppm de F1122 ($CF_2CHCl$)

*Principales impuretés saturées :*

60 ppm de F114a ($CCl_2FCF_3$)
370 ppm de F124 ($CHClFCF_3$)
250 ppm de F133a ($CH_2ClCF_3$)
545 ppm de F143a ($CH_3CF_3$)

La réaction a été réalisée à 200°C sous pression atmosphérique, le débit d'alimentation du mélange HF/F134a étant ajusté de façon à maintenir un temps de contact de 80 secondes. Afin de suivre l'évolution du taux d'oléfines dans les produits organiques, un échantillon gazeux prélevé en sortie de réacteur a été analysé par CPV, après élimination des hydracides (HF principalement) par lavage et séchage sur chlorure de calcium. On a obtenu les résultats suivants :

*Taux d'oléfines en sortie de réacteur :*

40 ppm de F1243 + F1234 non séparés
< 1 ppm de F1122 (limite de détection)

Ces résultats montrent que les oléfines en $C_3$ (F1234 et F1243) sont nettement moins réactives vis-à-vis de l'HF que le F1122 et qu'il est pratiquement impossible de les éliminer entièrement par une simple fluoration.

***(c) <u>Purification du F134a par Cl2 sans HF</u>***

Dans le même réacteur et avec le même catalyseur, on a fait passer un mélange $Cl_2$/F134a dans les conditions suivantes :

| | |
|---|---|
| Pression | atmosphérique |
| Temps de contact | 80 secondes |
| Rapport molaire $Cl_2$/F134a | 0,007 |
| Température | 200°C |

Le F134a utilisé contenait les impuretés suivantes :

*Impuretés oléfiniques* :

63 ppm de F1243 + F1234 ($C_3H_3F_3$ + $C_3H_2F_4$) non séparés
14 ppm de F1225 ($C_3HF_5$)
180 ppm de F1122 ($CF_2CHCl$)

*Principales impuretés saturées :*

30 ppm de F124 ($CHFClCF_3$)
39 ppm de F133a ($CH_2ClCF_3$)

Les résultats suivants ont été obtenus :

*Taux d'oléfines en sortie de réacteur :*

| | |
|---|---|
| F1234 + F1243 | < 1 ppm (limite de détection) |
| F1225 | < 1 ppm (limite de détection) |
| F1122 | < 1 ppm (limite de détection) |

*Taux de F133a, F123, F123a et F124 en sortie de réacteur :*

| | |
|---|---|
| F123 + F123a | 0,085 % |
| F124 | 0,134 % |
| F133a | 0,270 % |

Ces résultats montrent que l'élimination des oléfines en $C_3$ par chloration est possible, mais s'accompagne d'une perte de rendement par formation de F124 et de F133a.

***(d) Purification du F134a par HF + Cl2***

Dans le même réacteur et avec le même catalyseur que ci-dessus on a fait passer un mélange $Cl_2$/HF/F134a dans les conditions suivantes :

| | |
|---|---|
| Pression | atmosphérique |
| Temps de contact | 80 secondes |
| Rapport molaire HF/F134a | 0,2 |
| Rapport molaire $Cl_2$/F134a | 0,004 à 0,018 |
| Température | 150 à 225°C |

Le F134a brut utilisé était le même que celui de l'essai (c) précédent.

La même charge de catalyseur a été utilisée pendant 130 heures pour effectuer les 6 essais résumés dans le tableau suivant, la durée de chaque essai étant comprise entre 19 et 24 heures.

Les résultats suivants ont été obtenus :

| **Température** | **Rapport molaire** | **Taux d'oléfines en sortie de réacteur (ppm)** | | **Taux de F133a, F123, F123a et F124 en sortie de réacteur (%)** | | |
|---|---|---|---|---|---|---|
| (°C) | $Cl_2$/F134a | F1234 + F1243 | F1225 | F123 + F123a | F124 | F133a |
| 150 | 0,018 | <1 | <1 | 0,020 | 0,006 | 0,002 |
| 150 | 0,010 | <1 | 4 | 0,016 | 0,004 | 0,007 |
| 150 | 0,004 | 2 | 7 | 0,010 | 0,003 | 0,008 |
| 200 | 0,009 | <1 | 2 | 0,013 | 0,004 | 0,014 |
| 200 | 0,005 | <1 | 4 | 0,014 | 0,003 | 0,033 |
| 225 | 0,004 | <1 | 3 | 0,025 | 0,020 | 0,082 |

Après purification, il ne restait plus de trace de F1122 (limite de détection : <1 ppm).

Ces résultats montrent que l'emploi d'un mélange $Cl_2$/HF permet, sur un catalyseur au nickel, de purifier efficacement le F134a. Ce traitement permet de supprimer les oléfines F1234, F1243 et la majorité du F1225, tout en limitant la dégradation du F134a (formation de F123, F123a, F124 et F133a).

**EXEMPLE 6**

En utilisant une solution d'imprégnation contenant 117 g de chlorure de nickel hexahydraté et 150 g d'eau et en opérant comme à l'exemple 5(a), on a préparé un catalyseur au nickel sur alumine fluorée présentant les caractéristiques physico-chimiques suivantes :

- *Composition chimique (pondérale)* :
  - fluor : 61,5 %
  - aluminium : 27,5 %
  - nickel : 8,25 %
- *Propriétés physiques* :

- densité apparente (en vrac) : 0,71 g/ml
- surface BET : 25 $m^2/g$
- volume des pores d'un rayon compris entre 4 nm et 63 µm : 0,52 ml/g
- surface des pores d'un rayon supérieur à 4 nm : 30,9 $m^2/g$

Dans le même réacteur qu'à l'exemple 5(b), on a placé 300 ml de ce catalyseur et on y a fait passer un mélange $Cl_2$/HF/134a dans les conditions suivantes :

| | |
|---|---|
| Pression | atmosphérique |
| Temps de contact | 80 secondes |
| Rapport molaire HF/F134a | 0,2 |
| Rapport molaire $Cl_2$/F134a | 0,005 |
| Température | 200°C |

Le F134a brut utilisé était le même que celui de l'essai 5(c).
Après 19 heures de test, les résultats suivants ont été obtenus :

*Taux d'oléfines en sortie de réacteur :*

| | |
|---|---|
| F1234 + F1243 | < 1 ppm |
| F1225 | < 1 ppm |
| F1122 | < 1 ppm |

*Taux de F133a, F123, F123a et F124 en sortie de réacteur :*

| | |
|---|---|
| F123 + F123a | 0,015 % |
| F124 | 0,011 % |
| F133a | 0,048 % |

L'essai a été prolongé et, après 130 heures de marche, le taux résiduel en oléfines ainsi que les taux de F133a, F123, F123a et F124 formés étaient identiques.

## EXEMPLE 7

En opérant comme décrit dans la demande de brevet EP 0 486 333 à partir de chlorure de nickel, d'oxyde de chrome et d'alumine partiellement fluorée, on a préparé un catalyseur qui, après activation en lit fixe par un mélange azote/HF, présente les caractéristiques physico-chimiques suivantes :

- *Composition chimique (pondérale)* :
  - fluor : 56,9 %
  - aluminium : 25,5 %
  - nickel : 6,2 %
  - chrome : 6,2 %
  - chlore : 0,7 %
- *Propriétés physiques* :
  - densité apparente (en vrac) : 0,84 g/ml
  - surface BET : 59,1 $m^2/g$
  - volume des pores d'un rayon compris entre 4 nm et 63 µm : 0,37 ml/g
  - surface des pores d'un rayon supérieur à 4 nm : 31 $m^2/g$

Dans le même réacteur qu'à l'exemple 5(b), on a placé 300 ml de ce catalyseur et on y a fait passer un mélange

$Cl_2$/HF/134a dans les conditions suivantes :

| | |
|---|---|
| Pression | atmosphérique |
| Temps de contact | 80 secondes |
| Rapport molaire HF/F134a | 0,2 |
| Rapport molaire $Cl_2$/F134a | 0 à 0,01 |
| Température | 150°C à 225°C |

Le F134a utilisé contenait les impuretés suivantes :

*Impuretés oléfiniques :*

59 ppm de F1243 + F1234 ($C_3H_3F_3$ + $C_3H_2F_4$) non séparés
14 ppm de F1225 ($C_3HF_5$)
232 ppm de F1122 ($CF_2CHCl$)

*Principales impuretés saturées :*

30 ppm de F124 ($CHClFCF_3$)
39 ppm de F133a ($CH_2ClCF_3$)

La même charge de catalyseur a été utilisée pour effectuer l'ensemble des essais résumés dans le tableau suivant, la durée de chaque essai étant comprise entre 19 et 24 heures.

| **Température** | **Rapport molaire** | **Taux d'oléfines en sortie de réacteur (ppm)** | | **Taux de F133a, F123, F123a et F124 en sortie de réacteur (%)** | | |
|---|---|---|---|---|---|---|
| (°C) | $Cl_2$/F134a | F1234 + F1243 | F1225 | F123 + F123a | F124 | F133a |
| 150 | 0,010 | <1 | 4 | 0,033 | 0,005 | 0,007 |
| 150 | 0,005 | 2 | 5 | 0,027 | 0,004 | 0,009 |
| 150 | 0 | 58 | 14 | 0 | 0,003 | 0,033 |
| 200 | 0,005 | <1 | 5 | 0,016 | 0,005 | 0,030 |
| 225 | 0,004 | <1 | 3 | 0,012 | 0,009 | 0,150 |

Après purification, il ne restait plus de traces de F1122 (< 1 ppm).

## Revendications

1. Procédé de purification d'un 1,1,1,2-tétrafluoroéthane (F134a) brut contenant des impuretés insaturées, caractérisé en ce que l'on traite en phase gazeuse, un mélange gazeux de 1,1,1,2-tétrafluoroéthane brut, d'acide fluorhydrique et de chlore à une température comprise entre 100 et 300°C et sous une pression allant de la pression atmosphérique jusqu'à 2,5 MPa, en présence d'un catalyseur de fluoration, le rapport molaire HF/F134a étant compris entre 0,05 et 0,5 et le rapport molaire $Cl_2$/F134a étant compris entre 0,0001 et 0,1.

2. Procédé selon la revendication 1 dans lequel les impuretés insaturées sont le 1-chloro-2,2-difluoroéthylène et/ou des oléfines (chloro)fluorées en $C_3$ ou $C_4$.

3. Procédé selon la revendication 1 ou 2, dans lequel on opère sous une pression comprise entre la pression atmosphérique et 1,5 MPa.

4. Procédé selon l'une des revendications 1 à 3, dans lequel le temps de contact est compris entre 10 et 200 secondes, de préférence entre 20 et 100 secondes.

5. Procédé selon l'une des revendications 1 à 4, dans lequel le rapport molaire HF/F134a est compris entre 0,125

et 0,300.

6. Procédé selon l'une des revendications 1 à 5, dans lequel le rapport molaire $Cl_2$/F134a est compris entre 0,001 et 0,03.

7. Procédé selon l'une des revendications 1 à 6, dans lequel le catalyseur de fluoration est un catalyseur massique ou supporté à base de chrome, nickel, fer, manganèse, cobalt et/ou zinc.

8. Procédé selon la revendication 7, dans lequel on utilise comme catalyseur un oxyde de chrome massique.

9. Procédé selon la revendication 7, dans lequel on utilise comme catalyseur un fluorure de nickel supporté sur alumine fluorée.

10. Procédé selon la revendication 7, dans lequel on utilise un catalyseur à base de fluorure de nickel et d'oxyde de chrome supportés sur fluorure d'aluminium.

**Patentansprüche**

1. Verfahren zur Reinigung von Roh-1,1,1,2-tetrafluorethan (F134a), enthaltend ungesättigte Verunreinigungen, dadurch gekennzeichnet, daß man in der Gasphase eine gasförmige Mischung von Roh-1,1,1,2-tetrafluorethan, Fluorwasserstoff und Chlor bei einer Temperatur zwischen 100 und 300 °C und unter einem Druck, der von Atmosphärendruck bis 2,5 MPa reicht, in Gegenwart eines Fluorierungskatalysators behandelt, wobei das Molverhältnis von HF/F134a zwischen 0,05 und 0,5 liegt und das Molverhältnis von $Cl_2$/F134a zwischen 0,0001 und 0,1 liegt.

2. Verfahren nach Anspruch 1, bei dem die ungesättigten Verunreinigungen 1-Chlor-2,2-difluorethylen und/oder (chlor)fluorierte $C_3$-$C_4$-Olefine sind.

3. Verfahren nach Anspruch 1 oder 2, bei dem man unter einem Druck zwischen Atmosphärendruck und 1,5 MPa verfährt.

4. Verfahren nach einem der Ansprüche 1 bis 3, bei dem die Kontaktzeit zwischen 10 und 200 Sekunden, vorzugsweise zwischen 20 und 100 Sekunden, liegt.

5. Verfahren nach einem der Ansprüche 1 bis 4, bei dem das Molverhältnis von HF/F134a zwischen 0,125 und 0,300 liegt.

6. Verfahren nach einem der Ansprüche 1 bis 5, bei dem das Molverhältnis von $Cl_2$/F134a zwischen 0,001 und 0,03 liegt.

7. Verfahren nach einem der Ansprüche 1 bis 6, bei dem der Fluorierungskatalysator ein Katalysator in Masse oder ein auf einem Trägermaterial aufgebrachter Katalysator auf Basis von Chrom, Nickel, Eisen, Mangan, Kobalt und/oder Zink ist.

8. Verfahren nach Anspruch 7, bei dem man als Katalysator ein Chromoxid in Masse verwendet.

9. Verfahren nach Anspruch 7, bei dem man als Katalysator ein Nickelfluorid verwendet, das auf fluoriertes Aluminiumoxid aufgebracht ist.

10. Verfahren nach Anspruch 7, bei dem man einen Katalysator auf Basis von Nickelfluorid und Chromoxid verwendet, die auf Aluminiumfluorid aufgebracht sind.

**Claims**

1. Process for the purification of a crude 1,1,1,2-tetrafluoroethane (F134a) containing unsaturated impurities, characterized in that a gaseous mixture of crude 1,1,1,2-tetrafluoroethane, hydrofluoric acid and chlorine is treated in the gas phase, at a temperature between 100 and 300°C and at a pressure ranging from atmospheric pressure

to 2.5 MPa, in the presence of a fluorination catalyst, the HF/F134a molar ratio being between 0.05 and 0.5 and the $Cl_2$/F134a molar ratio being between 0.0001 and 0.1.

**2.** Process according to Claim 1, in which the unsaturated impurities are l-chloro-2,2-difluoroethylene and/or $C_3$ or $C_4$ (chloro)fluorinated olefins.

**3.** Process according to Claim 1 or 2, in which the reaction is carried out at a pressure between atmospheric pressure and 1.5 MPa.

**4.** Process according to one of Claims 1 to 3, in which the contact time is between 10 and 200 seconds, preferably between 20 and 100 seconds.

**5.** Process according to one of Claims 1 to 4, in which the HF/F134a molar ratio is between 0.125 and 0.300.

**6.** Process according to one of Claims 1 to 5, in which the $Cl_2$/F134a molar ratio is between 0.001 and 0.03.

**7.** Process according to one of Claims 1 to 6, in which the fluorination catalyst is a bulk or supported catalyst based on chromium, nickel, iron, manganese, cobalt and/or zinc.

**8.** Process according to Claim 7, in which a bulk chromium oxide is used as catalyst.

**9.** Process according to Claim 7, in which a nickel fluoride supported on fluorinated alumina is used as catalyst.

**10.** Process according to Claim 7, in which a catalyst based on nickel fluoride and on chromium oxide which are supported on aluminium fluoride is used.